# EUROPEAN PATENT APPLICATION

(11) **EP 1 541 692 A1**
(43) Date of publication of application: **15.06.2005**
(21) Application number: 03794104.4
(22) Date of filing: 27.08.2003
(51) Int. Cl.: C12P 17/14, C12P 41/00, C07C 319/14, C07C 319/06, C07C 323/58, C07D 233/76, C07B 53/00

(54) **PROCESS FOR PRODUCING L-a-METHYLCYSTEINE DERIVATIVE**

(30) Priority: 06.09.2002 JP 2002261524
(71) Applicant: KANEKA CORPORATION, Osaka-shi, Osaka 530-8288 (JP)
(72) Inventor: OHISHI, Takahiro, c/o KANEKA CORPORATION, Takasago-shi, Hyogo 676-8688 (JP); NANBA, Hirokazu, c/o KANEKA CORPORATION, Takasago-shi, Hyogo 676-8688 (JP); SUGAWARA, Masanobu, c/o KANEKA CORPORATION, Takasago-shi, Hyogo 676-8688 (JP); IZUMIDA, Masashi, c/o KANEKA CORPORATION, Takasago-shi, Hyogo 676-8688 (JP); HONDA, Tatsuya, c/o KANEKA CORPORATION, Takasago-shi, Hyogo 676-8688 (JP); MORI, Kouhei, c/o KANEKA CORPORATION, Takasago-shi, Hyogo 676-8688 (JP); YANAGISAWA, Satohiro, c/o KANEKA CORPORATION, Takasago-chi, Hyogo 676-8688 (JP); INOUE, Kenji, c/o KANEKA CORPORATION, Takasago-shi, Hyogo 676-8688 (JP)
(74) Representative: HOFFMANN - EITLE
(86) International application number: PCT/JP2003/010881
(87) International publication number: WO 2004/022766

(57) **Abstract**

A process for easily producing an L-α-methylcysteine derivative or its salt, which is useful as a drug intermediate, from a cheap easily procurable raw material through an enzymatic D-stereoselective hydrolysis of racemic 5-halomethyl-5-methyl-hydantoin. L-α-methylcysteine derivative or its salt is produced by converting racemic 5-halomethyl-5-methylhydantoin to L-5-halomethyl-5-methylhydantoin through an enzymatic D-stereoselective hydrolysis, reacting the L-5-halomethyl-5-methylhydantoin with a sulfurizing agent into L-5-methyl-5-thiomethylhydantoin and hydrolyzing the L-5-methyl-5-thiomethylhydantoin.

## Description

### Technical Field

The present invention relates to L-α-methylcysteine derivatives or their salts, which are useful as drug intermediates, and a process for producing the same. The present invention further relates to L-5-halomethyl-5-methylhydantoins and L-5-methyl-5-thiomethylhydantoin, which are intermediates of the L-α-methylcysteine derivatives, and a process for producing the same.

### Background Art

L-α-Methylcysteine derivatives or salts thereof have been produced by the following known methods:
(1) Asymmetric alkylation of an optically active thiazolidine compound which is prepared from optically active cysteine and pivalaldehyde (PCT Japanese Translation Patent Publication No. 2000-515166, International Publication Nos. WO01/72702 and WO01/72703 (US patent Nos. 6,403,830 and 6,586,474, and European Patent Nos. EP1265859A2 and EP1265860A1)),
(2) Asymmetric thioalkylation of an optically active thiazolidine compound that is prepared from optically active alanine and benzaldehyde (Tetrahedron, 1999, 55:10685-10694),
(3) Asymmetrical bromomethylation of an optically active diketopiperazine compound synthesized from optically active valine and alanine, and then replacing a bromine atom of the resulting compound with an alkali metal alkylthiolate (J. Org. Chem., 1992, 57:5568-5573),
(4) Synthesis of an optically active aziridine compound from optically active 2-methylglycidol prepared by Sharpless asymmetric oxidation of 2-methyl-2-propen-1-ol followed by reaction with thiol (J. Org. Chem., 1995, 60:790-791),
(5) Desymmetrization of an alkylated aminomalonic acid derivative with pig liver esterase (hereinafter abbreviated to PLE), and then reacting the resulting optically active ester with an alkali metal salt of thioacetic acid (J. Am. Chem. Soc., 1993, 115:8449-8450), and
(6) Separation and purification of a racemic thiazoline compound, which is prepared by methylation of a thiazoline compound synthesized from a cysteine derivative, by chiral high-performance liquid chromatography (HPLC) (Synlett., 1994, 9:702-704).

Any method disclosed in (1), (2), and (3) includes a low-temperature reaction using an expensive base such as butyllithium and requires a special manufacturing facility. The method described in (4) is complicated due to a large number of steps. This is disadvantageous for industrial application. In the method described in (5), desymmetrization of a diester using PLE is a key step. PLE is unsuitable for mass production and cannot be produced on an industrial scale. Therefore, method (5) is not practical. In the method described in (6), since an asymmetric alkylation process of the thiazoline compound is not known, the racemic compounds have to be separated by chiral HPLC. Furthermore, since the unnecessary enantiomer cannot be racemized, it cannot be reused. Therefore, the productivity of this method is low and the preparation by this method on an industrial scale is disadvantageous. When these methods are applied as an industrial manufacturing process to prepare optically active α-substituted cysteine or its salt, all the above methods have problems to be solved.

A possible process for effectively preparing an L-α-methylcysteine derivative or its salt includes the steps of stereoselectively hydrolyzing a 5-halomethyl-5-methylhydantoin, which is easily obtained from a haloacetone, to an optically active L-5-halomethyl-5-methylhydantoin with hydantoinase; synthesizing an L-5-methyl-5-thiomethylhydantoin by reaction of the resulting L-5-halomethyl-5-methylhydantoin with a sulfurizing agent, and then hydrolyzing the resulting L-5-methyl-5-thiomethylhydantoin. However, no process is known to effectively prepare an optically active hydantoin having two different substituents at the 5-position by stereoselective hydrolysis with hydantoinase, nor for preparing an optically active amino acid derivative having two different substituents at the α-position by hydrolysis of the resulting hydantoin.

Accordingly, it is an object of the present invention to provide a method for readily preparing L-α-methylcysteine and its salt, which is useful as a drug intermediate, from a cheap and readily available material by D-stereoselective hydrolysis with a racemic 5-halomethyl-5-methylhydantoin and hydantoinase.

### Summary of the Invention

In view of above, the inventors have intensively studied in order to find a method for preparing an L-α-methylcysteine derivative or its salt by D-stereoselectively hydrolyzing a 5-halomethyl-5-methylhydantoin, which can be easily prepared using a cheap and readily available haloacetone, to produce an optically active L-5-halomethyl-5-methylhydantoin by hydantoinase; synthesizing an L-5-methyl-5-thiomethylhydantoin by the reaction of the L-5-halomethyl-5-methylhydantoin with a sulfurizing agent; and then hydrolyzing the resulting L-5-methyl-5-thiomethylhydantoin, and, as a result, have completed the present invention.

The present invention relates to a method for preparing an L-5-halomethyl-5-methylhydantoin represented by Formula (2): 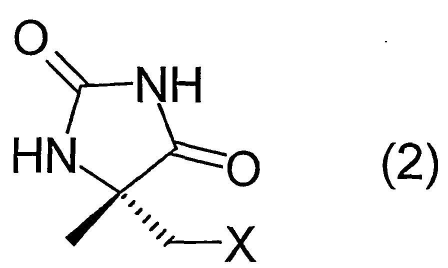 (wherein X denotes a halogen atom), by D-stereoselectively hydrolyzing a racemic 5-halomethyl-5-methylhydantoin represented by Formula (1): 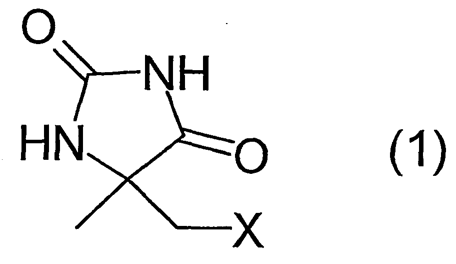 (wherein X is the same as above), with hydantoinase.

The present invention is also relates to a method for preparing an L-5-methyl-5-thiomethylhydantoin represented by Formula (3): 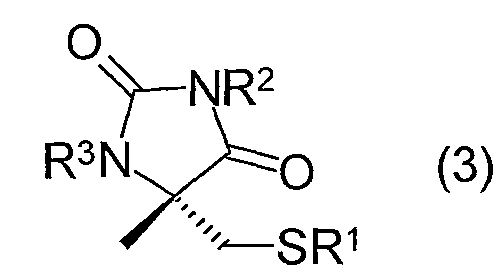 (wherein R¹ denotes a hydrogen atom, an alkyl group having 1 to 20 carbon atoms which may be linear, branched, or cyclic, a substituted or unsubstituted benzyl group, an alkanoyl group having 1 to 20 carbon atoms, or an alkoxycarbonyl group having 1 to 20 carbon atoms; and each of R² and R³ independently denotes a hydrogen atom, or an alkanoyl group having 1 to 20 carbon atoms, and may be the same or different), by reacting an L-5-halomethyl-5-methylhydantoin represented by Formula (2) with a sulfurizing agent.

Furthermore, the present invention relates to a method for preparing an L-α-methylcysteine derivative or its salt represented by Formula (4): 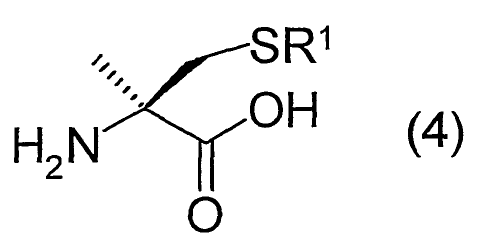 (wherein R¹ is the same as above), by hydrolyzing an L-5-methyl-5-thiomethylhydantoin represented by Formula (3) with an acid or alkali and by optionally deprotecting a nitrogen atom and/or a sulfur atom.

Furthermore, the present invention provides an L-5-halomethyl-5-methylhydantoin represented by Formula (2): 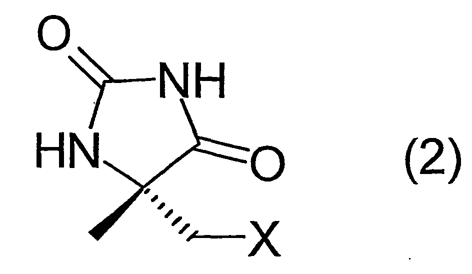 (wherein X is the same as above).

The present invention also provides an L-5-methyl-5-thiomethylhydantoin represented by Formula (5): 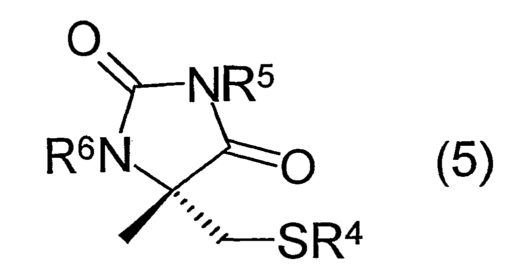 (wherein R⁴ denotes an alkyl group having 1 to 20 carbon atoms which may be linear, secondary, or cyclic, a substituted or unsubstituted benzyl group, an alkanoyl group having 1 to 20 carbon atoms, or an alkoxycarbonyl group having 1 to 20 carbon atoms; and each of R⁵ and R⁶ independently denotes a hydrogen atom or an alkanoyl group having 1 to 20 carbon atoms and may be the same or different).

Furthermore, the present invention provides an L-α-methylcysteine derivative or its salt represented by Formula (6): 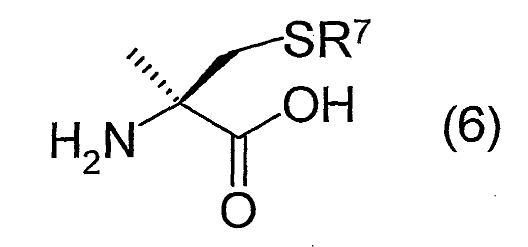 (wherein R⁷ is a substituted or unsubstituted benzyl group, an alkanoyl group having 1 to 20 carbon atoms, or an alkoxycarbonyl group having 1 to 20 carbon atoms).

### Detailed Description of the Invention

The present invention will now be described in detail.

In the present invention, an L-5-halomethyl-5-methylhydantoin represented by Formula (2) is prepared by D-stereoselectively hydrolyzing a racemic 5-halomethyl-5-methylhydantoin represented by Formula (1) using hydantoinase.

The racemic 5-halomethyl-5-methylhydantoin (1) can be readily prepared by, for example, a Bucherer reaction. Namely, a target hydantoin is synthesized by stirring a corresponding haloketone with sodium cyanide or potassium cyanide and ammonium carbonate in a solvent mixture of water and ethanol.

Hydantoinase used in this process is an enzyme having an activity that generates an N-carbamoyl-α-amino acid derivative by hydrolysis of a 5-substituted hydantoin derivative. Hydantoinase used in the present invention to catalyze the stereoselective hydrolysis can be derived from animals, plants, or microorganisms. An enzyme derived from microorganisms is preferable in terms of industrial application. Any microorganism that is capable of generating such an enzyme can be utilized, including the following known microorganisms:
Examples of such microorganisms belonging to bacteria include Acetobacter, Achromobacter, Aerobacter, Agrobacterium, Alcaligenes, Arthrobacter, Bacillus, Brevibacterium, Corynebacterium, Enterobacter, Erwinia, Escherichia, Klebsiella, Microbacterium, Micrococcus, Protaminobacter, Proteus, Pseudomonas, Sarcina, Serratia, Xanthomonas, Aeromonas, Flavobacterium, and Rhizobium.
Examples of microorganisms belonging to actinomycetes include Actinomyces, Mycobacterium, Nocardia, Streptomyces, Actinoplanes, and Rhodococcus.
Examples of microorganisms belonging to molds include Aspergillus, Paecilomyces, and Penicillium.
Examples of microorganisms belonging to yeasts include Candida, Pichia, Rhodotorula, and Torulopsis.

Enzymes derived from microorganisms belonging to Agrobacterium, Bacillus, Pseudomonas, and Rhizobium are preferable.

Enzymes derived from Agrobacterium sp. strain KNK712 (FERM BP-1900), Bacillus sp. strain KNK245 (FERM BP-4863), Pseudomonas putida IFO 12996, Pseudomonas sp. strain KNK003A (FERM BP-3181), and Rhizobium sp. strain KNK1415 (FERM BP-4419) are more preferable.

In the present invention, the hydantoinase can be used in the forms of the enzyme itself, a microorganism or its processed product having the enzyme activity, or a transformed microorganism or its processed product having the enzyme activity.

Examples of the processed product of the microorganism include a crude extract, a lyophilized organism, an acetone-dried organism, and disrupted cells thereof. Furthermore, the enzyme itself or cells may be immobilized by a known method. The immobilization can be conducted by a process widely known by those skilled in the art, for example, crosslinking, covalent binding, ionic binding, physical adsorption, or an entrapment method. Examples of a preferable support for the enzyme immobilization include phenol-formaldehyde anion-exchange resins such as Duolites A568 and DS17186 (trademark of Rohm and Haas Company); and a variety of anion-exchange resins based on polystyrene resins having functional groups of various amines, ammonium salts, or diethanolamines, such as Amberlites IRA935, IRA945, and IRA901 (trademark of Rohm and Haas Company), Lewatit OC1037 (trademark of Bayer Corporation), and Diaion EX-05 (trademark of Mitsubishi Chemical Corporation). DEAE-cellulose can be also used as the support.

The immobilized enzyme may be adequately prepared by, for example, a method described in International Publication No. WO96/20275, i.e. harvesting cells showing hydantoinase activity from a culture, disrupting the harvested cells by sonication or the like, adding an anion-exchange resin, for example, Duolite A-568 to the resulting enzyme solution, and then stirring the solution in order to adsorb the enzyme. The resin adsorbed with the enzyme may be additionally stabilized by crosslinking, i.e. stirring the resin with a crosslinking reagent such as glutaraldehyde. Following these processes, the resin is then collected by filtration, and washed in order to prepare the immobilized hydantoinase.

The production of transformed microorganisms is effective in order to prepare highly active microorganism that effectively produce hydantoinase. A method for preparing the transformed microorganisms is described in, for example, International Publication No. WO96/20275, i.e. cloning a gene encoding the hydantoinase from a microorganism strain showing the hydantoinase activity, generating an appropriate recombinant plasmid vector, and then transforming appropriate host cells with the vector. The recombinant DNA technology is widely known in the field. See, for example, Molecular Cloning 2nd Edition (Cold Spring Harbor Laboratory Press, 1989), Current Protocols in Molecular Biology (Greene Publishing Associates and Wiley-Interscience).

Examples of the transformed microorganism effectively producing hydantoinase prepared as described above include Escherichia coli HB101 pTH104 (FERM BP-4864) containing a gene encoding hydantoinase derived from Bacillus sp. strain KNK245 (FERM BP-4863), Escherichia coli HB101 pAH1043 (FERM BP-4865) containing a gene encoding hydantoinase derived from Agrobacterium sp. strain KNK712 (FERM BP-1900), and Escherichia coli HB101 pPHD301 (FERM BP-4866) containing a gene encoding hydantoinase derived from Pseudomonas sp. strain KNK003A (FERM BP-3181), which are disclosed in International Publication No. WO96/20275.

Hydantoinase can be produced by culturing these transformants or the above-mentioned microorganism strains showing hydantoinase activities in a commonly-used nutrient medium disclosed in, for example, International Publication No. WO96/20275. A treatment for enzyme induction may be conducted if necessary.

The enzyme reaction according to the present invention can be performed as described below. In the reaction using hydantoinase, a racemic 5-halomethyl-5-methylhydantoin represented by Formula (1) is used as a substrate in an aqueous solvent.

In the reaction, the substrate is dissolved or suspended at an initial loading concentration of 0.1% to 90%(w/v), the reaction temperature is appropriately adjusted in the range of 10°C to 80°C, the pH is maintained in the range of 4 to 10, and the solution is left to stand or stirred for a while. The substrate may be added continuously. The reaction can be carried out in either a batch or continuous process.

An immobilized enzyme, a membrane reactor, or the like can be used for the reaction according to the present invention. As mentioned above, the enzyme itself, the microorganism or its processed product having the enzyme activity, and the transformed microorganism or its processed product having the enzyme activity can be used as hydantoinase. Examples of the aqueous solvent include water, a buffer solution, and an aqueous solvent thereof containing a water-soluble organic solvent such as ethanol. Other examples include a suitable solvent consisting of two phases of an aqueous solvent and a water-insoluble organic solvent. Examples of the organic solvent include ethyl acetate, butyl acetate, toluene, chloroform, and n-hexane. Furthermore, an antioxidant, surfactant, coenzyme, metal, and the like may be added if necessary.

The resulting L-5-halomethyl-5-methylhydantoin can be used directly, without purification, in the next process. Preferably, it is purified by crystallization to improve the optical and chemical purities.

Examples of the solvent for the crystallization include ethyl acetate, methyl alcohol, ethyl alcohol, propyl alcohol, isopropyl alcohol, n-butyl alcohol, sec-butyl alcohol, tert-butyl alcohol, acetone, tetrahydrofuran, and acetonitrile. These solvents may be used alone or in combination. Among them, ethyl acetate and ethyl alcohol are preferable. In the crystallization, a poor solvent may be simultaneously used to increase the yield. Examples of the poor solvent include benzene, toluene, hexane, heptane, and cyclohexane. Hexane and heptane are preferable.

Next, a reaction process of an L-5-methyl-5-methylhydantoin represented by Formula (2) with a sulfurizing agent in order to prepare an L-5-methyl-5-thiomethyhydantoin represented by Formula (3) will be described.

In Formula (3), R¹ denotes a hydrogen atom; an alkyl group having 1 to 20 carbon atoms, which may be linear, branched, or cyclic; a substituted or unsubstituted benzyl group; or an alkanoyl group having 1 to 20 carbon atoms.

Examples of the alkyl group having 1 to 20 carbon atoms, which may be linear, branched, or cyclic include a methyl group, an ethyl group, a propyl group, an isopropyl group, an n-butyl group, a sec-butyl group, a tert-butyl group, a pentyl group, a hexyl group, a cyclopropyl group, a cyclobutyl group, a cyclopentyl group, and a cyclohexyl group. The tert-butyl group is preferable because of easy deprotection.

Examples of the substituted or unsubstituted benzyl group include a benzyl group, an o- or p-methoxybenzyl group, an o- or p-hydroxybenzyl group, an o- or p-acetoxybenzyl group, a p-nitrobenzyl group, a 2,4,6-trimethylbenzyl group, and a 2,4,6-trimethoxybenzyl group. The benzyl group and the p-methoxybenzyl group are preferable because of easy deprotection.

Examples of the alkanoyl group having 1 to 20 carbon atoms include an acetyl group, a propanoyl group, a butanoyl group, a pentanoyl group, and a hexanoyl group. The acetyl group is preferable because of easy introduction and deprotection.

The alkoxycarbonyl group having 1 to 20 carbon atoms may be any common protecting group for sulfur. A benzyloxycarbonyl group and a tert-butyloxycarbonyl group are preferable. Each of R² and R³ independently denotes a hydrogen atom or an alkanoyl group having 1 to 20 carbon atoms and may be the same or different.

Examples of the alkanoyl group having 1 to 20 carbon atoms include an acetyl group, a propanoyl group, a butanoyl group, a pentanoyl group, and a hexanoyl group. The acetyl group is preferable because of easy introduction and deprotection.

Examples of the sulfurizing agent used in the reaction include alkali metal hydrosulfides or alkaline-earth metal hydrosulfides such as sodium hydrosulfide, potassium hydrosulfide, calcium hydrosulfide, barium hydrosulfide, magnesium hydrosulfide, lithium hydrosulfide, and rubidium hydrosulfide; ammonium hydrosulfide; alkyl mercaptans such as tert-butyl mercaptan; aralkyl mercaptans such as benzyl mercaptan, p-methoxybenzyl mercaptan; and thioacyl compounds such as potassium thioacetate and thioacetic acid.

Sodium hydrosulfide and potassium hydrosulfide are preferable because they are inexpensive and readily available for industrial use, easy to handle, provide good yields, and do not require a deprotection process for the sulfur atom. When a thiocarbonic acid or its salt such as thioacetic acid and potassium thioacetate is used as the sulfurizing agent, an acyl group may be introduced to the nitrogen atom.

Preferably, this reaction is performed in the presence of a base. Examples of the base include sodium hydroxide, potassium hydroxide, potassium carbonate, and sodium carbonate.

A solvent is selected depending on the sulfurizing agent used for the reaction, but is not particularly limited. For example, when sodium hydrosulfide or potassium hydrosulfide is used, solvents selected from the group consisting of water; alcohols such as methyl alcohol, ethyl alcohol, n-butyl alcohol, tert-butyl alcohol, and isopropyl alcohol; and aprotic polar solvents such as dimethylsufoxide, diethylsulfoxide, and dimethylformamide are desirably used alone or in combination.

The resulting L-5-methyl-5-thiomethylhydantoin (3) can be used directly, without purification, in the next process. It may be purified by crystallization or the like to improve the chemical and optical purities.

The hydrolysis of the L-5-methyl-5-tiomethylhydantoin (3) will now be described.

The hydantoin moiety can be hydrolyzed in either an acid or alkaline solution. After the hydrolysis, L-α-methylcysteine or its salt is produced directly or by further deprotection of the sulfur atom, depending on R¹ in the hydantoin (3) and the conditions for the hydrolysis.

For example, when R¹ is a tert-butyl group, an additional step to deprotect the sulfur atom is required in an alkaline solution. However, when the hydrolysis is performed with hydrochloric acid, a de-tert-butylation of the sulfur atom simultaneously occurs so as to produce L-α-methylcysteine hydrochloride.

When R¹ is an acetyl group, the deacetylation at the sulfur atom simultaneously occurs with hydrolysis in both the acid and alkaline solutions to produce L-α-methylcysteine or its salt. When R¹ is a benzyl group, the deprotection with sodium or the like is necessary after the hydrolysis.

Examples of the acid used in the acid hydrolysis include, but are not limited to, hydrochloric acid, sulfuric acid, nitric acid, hydrobromic acid, and acetic acid. Preferably, they are used alone or in combination.

Examples of the alkali used in the alkaline hydrolysis include, but are not limited to, sodium hydroxide, barium hydroxide, lithium hydroxide, potassium hydroxide, calcium hydroxide, sodium carbonate, and potassium carbonate. Sodium hydroxide, potassium hydroxide, barium hydroxide, calcium hydroxide, and lithium hydroxide are especially preferable from the viewpoint of reaction yield.

Preferably, the reaction temperature for the hydrolysis is between 70°C and 180°C. When the reaction temperature is higher than the boiling point of the solvent, a sealed autoclave is preferably used. When the reaction temperature is too low, a very long reaction time that spans several days is required and a disulfide may be formed as a by-product. When the reaction temperature is too high, other by-products may be formed. Therefore, the hydrolysis is preferably carried out at a temperature of 90°C to 150°C.

The sulfurization of L-5-halomethyl-5-methylhydantoin and the hydrolysis in an alkaline solution can be carried out in a one-pot.

Examples of the alkaline solution include sodium hydroxide, barium hydroxide, lithium hydroxide, potassium hydroxide, calcium hydroxide, and cesium hydroxide. Sodium hydroxide, potassium hydroxide, and lithium hydroxide are preferable. In such a case, examples of the sulfurizing agent include, but are not limited to, benzyl mercaptan, p-methoxybenzyl mercaptan, n-butyl mercaptan, sec-butyl mercaptan, and propyl mercaptan. Benzyl mercaptan and tert-butyl mercaptan are preferable.

Preferably, the sulfurization temperature ranges between 0°C and 100°C, more preferably, between 20°C and 80°C. The temperature for the following hydrolysis preferably ranges between 70°C and 180°C, more preferably, between 90°C and 150°C.

When L-α-methylcysteine or its salt is prepared by hydrolysis of hydantoin (3), a disulfide may be produced as a by-product. In such a case, the disulfide can be converted to L-α-methylcysteine or its salt by utilizing a reducing agent.

Examples of the reducing agent include, but are not limited to as long as they can cleave the disulfide bond, metals such as zinc, tin, and magnesium; and phosphine compounds. Among them, triarylphosphine and trialkylphophine compounds are preferable because of their significant decomposition ability and ease of removal by an extraction process. Triphenylphosphine is most preferable because of easy handling and reduced cost.

### Best Mode for Carrying Out the Invention

The present invention will now be further described with reference to Examples, however, the present invention is not limited to these Examples.

### (Reference Example 1) Method for preparing 5-methyl-5-chloromethylhydantoin

Ammonium carbonate (753 g, 6.6 mol) and sodium cyanide (135 g, 2.75 mol) were dissolved in distilled water (730 mL) at room temperature, and ethanol (730 mL) was added. Chloroacetone (204 g, 2.2 mol) was then added. The solution was stirred for 10 minutes, and was further stirred overnight at 60°C. The reaction mixture was spontaneously cool to room temperature, the solvent was evaporated under reduced pressure to approximately half the volume of the original solution. The solution was adjusted to pH 12 by adding 30 wt% sodium hydroxide (approximately 400 g), and was then washed with toluene (1.5 L × 2). The solution was adjusted to pH 7 by adding concentrated hydrochloric acid (700 g) in an ice-cooled environment, and was extracted with ethyl acetate (2 L × 2). The resulting organic phase was dried over anhydrous magnesium sulfate and then concentrated under reduced pressure to yield a white solid (198.5 g). The results of the HPLC analysis [column: COSMOSIL 5C8 (Nacalai Tesque, Inc.), mobile phase: acetonitrile/10mM potassium dihydrogenphosphate = 30/70, flow rate: 0.8 mL/min, detection wavelength: 210 nm, column temperature: 40°C] showed that the white solid contained 192.5 g (1.2 mol) of the titled compound (a yield of 54%). The white solid was used, without purification, in the next process. ¹H NMR (300 MHz, D₂O), δ: 3.86 (d, 1H), 3.75 (d, 1H), 1.51 (s, 3H).

### (Example 1) Method for preparing L-5-chloromethyl-5-methylhydantoin

According to methods described in International Publication No. WO96/20275, cells were cultured and an immobilized enzyme was prepared. Bacillus sp. strain KNK245 (FERM BP-4863) was cultured, and bacterial cells were collected. An enzyme solution was prepared by sonicating the collected bacterial cells. The enzyme was adsorbed to a carrier, i.e. an anionic exchange resin, Duolite A-568, which was added to the enzyme solution in order to immobilize the enzyme, and was then crosslinked with glutaraldehyde to prepare immobilized hydantoinase.

Next, water (91 mL) and a 0.5 M manganese sulfate solution (0.18 mL) were added to the crude crystals of racemic 5-chloromethyl-5-methylhydantoin (9.1 g; 83.6 wt%) prepared in Reference Example 1, and the solution was adjusted to pH 8.7 with a 20 wt% sodium hydroxide solution. Immobilized hydantoinase (32 g by wet weight), prepared as in the above, was added to the solution, and then the solution was reacted with stirring for 25 hours at 45°C. During the reaction, the solution was maintained at pH 8.7 by adding the 20 wt% sodium hydroxide solution. After completing the reaction, the immobilized hydantoinase was filtrated and washed. The resulting filtrate was adjusted to pH 7 with 6 N hydrochloric acid, and then extracted twice with an equivalent volume of ethyl acetate. The resulting ethyl acetate phase was dried over anhydrous sodium sulfate (80 g), and concentrated to dryness under reduced pressure to yield 5-chloromethyl-5-methylhydantoin (3.35 g).

The results of the HPLC analysis [column: two CHIROBIOTIC T (Advanced Separation Technologies Inc.) columns were connected, mobile phase: water/ethyl alcohol = 9/1, flow rate: 0.3 mL/min, detection wavelength: 210 nm, column temperature: 40°C] showed a chemical purity of 91.8 wt% and an optical purity of 97.0% ee. The yield of hydantoin was 40.5%.

The resulting optically active hydantoin was in the L-form. This was identified by measuring the optical rotation of methylcysteine converted from the resulting optically active hydantoin as in Examples 8 to 24.

### (Example 2) Method for preparing L-5-chloromethyl-5-methylhydantoin

Agrobacterium sp. strain KNK712 (FERM BP-1900) was cultured in a solid medium (10 g/L polypeptone, 10 g/L meat extract, 5 g/L yeast extract, 15 g/L, pH 7.5) at 30°C for 48 hours. The bacteria on a platinum loop were inoculated into 50 mL of a liquid medium (10 g/L polypeptone, 10 g/L meat extract, 5 g/L yeast extract, pH 7.5) sterilized at 120°C for 15 minutes in a 500-mL Sakaguchi flask, and were incubated with shaking at 30°C for 24 hours. Then, 1 mL of the culture solution was inoculated into a liquid medium, prepared by addition of 1 g/L uracil and 20 mg/L manganese chloride to the above-mentioned liquid medium, and was then incubated with shaking at 30°C for 24 hours. Bacterial cells separated from the culture solution (15 mL) by centrifugation were suspended in 1.5 mL of 0.1 M carbonic acid buffer (pH 8.7), and then racemic 5-chloromethyl-5-methylhydantoin (45 mg) and a 0.1 M manganese sulfate solution (0.015 mL) were added. The mixture was adjusted and maintained at about pH 8.7 with a 10 N sodium hydroxide solution and was reacted with stirring for 26 hours at 40°C. The results of the HPLC analysis [column: COSMOSIL 5C8-MS (Nacalai Tesque, Inc.), mobile phase: acetonitrile/10mM potassium dihydrogenphosphate = 5/95, flow rate: 0.8 mL/min, detection wavelength: 210 nm, column temperature: 40°C] of the reaction solution showed that the rate of remaining hydantoin was 39%. Hydantoin in the reaction solution had an optical purity of 92.7% ee according to an HPLC analysis (the analytical conditions were as in Example 1). The retention time compared with the hydantoin prepared in Example 1 indicated the resulting hydantoin as being in the L-form.

### (Example 3) Method for preparing L-5-chloromethyl-5-methylhydantoin using a Pseudomonas genus

Pseudomonas putida IFO12996 was cultured in a solid medium (10 g/L polypeptone, 2 g/L yeast extract, 1 g/L magnesium sulfate heptahydrate, 15 g/L agar, pH 7.0) at 30°C for 24 hours. The bacteria on a platinum loop were inoculated into 100 mL of a liquid medium (20 g/L meat extract, 6 g/L glycerol, 1 g/L uracil, 2 g/L potassium dihydrogenphosphate, 1 g/L magnesium sulfate heptahydrate, 40 mg/L calcium chloride dihydrate, 20 mg/L ferrous sulfate heptahydrate, 20 mg/L manganese sulfate tetra- to hexahydrate, 20 mg/L copper sulfate pentahydrate, pH 5.5) sterilized at 120°C for 15 minutes in a 500-mL Sakaguchi flask, and were incubated with shaking for 24 hours at 30°C. Bacterial cells that were separated from the culture solution (15 mL) by centrifugation were suspended in 1.5 mL of 0.1 M carbonic acid buffer (pH 8.7), and then racemic 5-chloromethyl-5-methylhydantoin (15 mg) and 0.5 M manganese sulfate solution (0.003 mL) were added. The mixture was adjusted and maintained at about pH 9.0 with a 10 N sodium hydroxide solution and was reacted with stirring for 52 hours at 40°C. The results of the HPLC analysis (the analytical conditions were as in Example 2) of the reaction solution showed that the rate of remaining hydantoin was 70%. Hydantoin in the reaction solution had an optical purity of 26.7% ee according to an HPLC analysis (the analytical conditions were as in Example 1). The retention time compared with the hydantoin prepared in Example 1 indicated the resulting hydantoin as being in the L-form.

### (Example 4) Method for preparing L-5-chloromethyl-5-methylhydantoin using Bacillus genus

Dried stock bacterial cells of Bacillus sp. strain KNK245 (FERM BP-4863) were inoculated into 100 mL of a liquid medium (10 g/L polypeptone, 10 g/L meat extract, 5 g/L yeast extract, pH 7.5) sterilized at 120°C for 15 minutes in a 500-mL Sakaguchi flask, and were incubated with shaking at 45°C for 15 hours. Then, 2 mL of the culture solution was inoculated into a medium, prepared by addition of 1 g/L uracil and 20 mg/L manganese chloride to the above-mentioned liquid medium, and were then incubated with shaking at 45°C for 24 hours. Bacterial cells that were separated from the culture solution (15 mL) by centrifugation were suspended in 1.5 mL of a 0.1 M carbonic acid buffer (pH 8.7), and then racemic 5-chloromethyl-5-methylhydantoin (15 mg) and a 0.5 M manganese sulfate solution (0.003 mL) were added. The solution was adjusted and maintained at about pH 9.0 with a 10 N sodium hydroxide solution and was then reacted with stirring at 40°C for 52 hours. The results of the HPLC analysis (the analytical conditions were as in Example 2) of the reaction solution identified that the rate of remaining hydantoin was 74%. The HPLC analysis (the analytical conditions were as in Example 1) of hydantoin in the reaction solution identified an optical purity of 30.7% ee. The retention time compared with the hydantoin prepared in Example 1 indicated the resulting hydantoin as being in the L-form.

### (Example 5) Method for preparing L-5-chloromethyl-5-methylhydantoin using Rhizobium genus

Dried stock bacterial cells of Rhizobium sp. strain KNK1415 (FERM BP-4419) were inoculated into 100 mL of a liquid medium (10 g/L polypeptone, 10 g/L meat extract, 5 g/L yeast extract, pH 7.5) sterilized at 120°C for 15 minutes in a 500-mL Sakaguchi flask, and were incubated with shaking at 30°C for 18 hours. Then, 1 mL of the culture solution was inoculated into a medium, prepared by addition of 1 g/L uracil and 20 mg/L manganese chloride to the above-mentioned liquid medium, and was then incubated with shaking at 30°C for 24 hours. Bacterial cells that were separated from the culture solution (15 mL) by centrifugation were suspended in 1.5 mL of a 0.1 M carbonic acid buffer (pH 8.7), and then racemic 5-chloromethyl-5-methylhydantoin (15 mg) and a 0.5 M manganese sulfate solution (0.003 mL) were added. The solution was then adjusted and maintained at about pH 9.0 with a 10 N sodium hydroxide solution and was reacted with stirring at 40°C for 32 hours. The results of the HPLC analysis (the analytical conditions were as in Example 2) of the reaction solution identified that the rate of remaining hydantoin was 54%. Furthermore, hydantoin with an optical purity of 22.5% ee was identified in the reaction solution by the HPLC analysis [column: two CHIROBIOTIC T (Advanced Separation Technologies Inc.) columns were connected, mobile phase: 0.036 M potassium dihydrogenphosphate-phosphoric acid solution (pH 2.2), flow rate: 0.5 mL/min, detection wavelength: 210 nm, column temperature: 40°C]. The retention time compared with the hydantoin prepared in Example 1 indicated the resulting hydantoin as being in the L-form.

### (Example 6) Method for preparing L-5-chloromethyl-5-methylhydantoin using Pseudomonas sp. strain KNK003A (FERM BP-3181)

Dried stock bacterial cells of Pseudomonas sp. strain KNK003A (FERM BP-3181) were inoculated into 100 mL of a liquid medium (10 g/L glycerin, 5 g/L glucose, 0.3 g/L yeast extract, 3.5 g/L potassium dihydrogenphosphate, 3.5 g/L disodium hydrogenphosphate, 0.5 g/L magnesium sulfate heptahydrate, 0.02 g/L manganese chloride tetrahydrate, 0.01 g/L iron sulfate heptahydrate, 1 g/L calcium carbonate, 3 g/L N-carbamoyl-D-alanine, pH 7.5, wherein glucose and N-carbamoyl-D-alanine were sterilized by filtration and then added to other constituents which were sterilized by heating) sterilized at 120°C for 15 minutes in a 500-mL Sakaguchi flask, and were incubated with shaking at 45°C for 46 hours. Bacterial cells that were separated from the culture solution (40 mL) by centrifugation were suspended in 1.5 mL of a 0.1 M carbonic acid buffer (pH 8.7), and then racemic 5-chloromethyl-5-methylhydantoin (15 mg) and a 0.5 M manganese sulfate solution (0.003 mL) were added. The solution was adjusted and maintained at about pH 9.0 with a 10 N sodium hydroxide solution and was reacted with stirring at 45°C for 24 hours. The results of the HPLC analysis (the analytical conditions were as in Example 2) of the reaction solution identified that the rate of remaining hydantoin was 54%. Hydantoin with an optical purity of 65.8% ee in the solution was identified by the HPLC analysis (the analytical conditions were as in Example 5). The retention time compared with the hydantoin prepared in Example 1 indicated the resulting hydantoin as being in the L-form.

### (Example 7) Method for purifying L-5-methyl-5-chloromethylhydantoin

Crude L-5-methyl-5-chloromethylhydantoin (68.85 g) prepared as in Example 1 was dissolved in ethyl alcohol (137.7 mL) and was then heated to 60°C. Hexane was slowly dropped until any white turbidity disappeared, while the internal temperature of the solution was controlled so as not to reach a temperature lower than 60°C. The amount of hexane used was 105 mL. Then, the solution was gradually cooled to -20°C and was stirred at -20°C overnight. Precipitated white crystals were separated by filtration and washed with 250 g of a hexane solution containing 5 wt% methyl alcohol to obtain the titled compound with a yield of 94.7%, a chemical purity of 94.6%, and an optical purity of 98.3% ee (the analytical conditions were as in Reference Example 1).

### (Example 8) Method for preparing L-5-mercaptomethyl-5 methylhydantoin

L-5-Chloromethyl-5-methylhydantoin (15.0 g, a chemical purity of 87 wt%, an optical purity of 98.3% ee, 80 mmol) and sodium hydrosulfide (60 g, a purity of 70 wt%) were dissolved in distilled water (150 g) and the solution was refluxed under nitrogen atmosphere for 1.5 hours. The solution was spontaneously cooled to room temperature and then adjusted to pH 1 with concentrated hydrochloric acid in an ice-cooled environment. The solution was extracted with ethyl acetate (500 mL × 4). The resulting organic phase was dried over anhydrous sodium sulfate and then concentrated under reduced pressure to yield a white solid (14.8 g). Analysis by HPLC [column: COSMOSIL C18-AR (Nacalai Tesque, Inc.), mobile phase: potassium dihydrogenphosphate-phosphoric acid solution (pH 2.0)/acetonitrile = 97/3, flow rate: 1.0 mL/min, detection wavelength: 210 nm, column temperature: 30°C] showed that the white solid contained 11.1 g (64.5 mmol) of the titled compound (a chemical purity of 75.0%, a yield of 80.7%).
¹H NMR (300 MHz, D₂O), δ: 3.52 (d, 1H), 3.32 (d, 1H), 1.40 (s, 3H).

### (Example 9) Method of purifying L-5-mercaptomethyl-5-methylhydantoin

L-5-Mercaptomethyl-5-chloromethylhydantoin (2.04 g, a chemical purity of 83.6%, an optical purity of 96.1% ee) was dissolved in ethyl acetate (40 mL) at 60°C. Hexane was slowly dropped until the white turbidity disappeared. The amount of hexane used was 90 mL. Then, the solution was gradually cooled to 0°C and was stirred at 0°C for 1 hour. Precipitated white crystals were separated by filtration and were washed with hexane (50 mL) to obtain the titled compound with a yield of 92.4%, a chemical purity of 91.3%, and an optical purity of 98.6% ee. The analytical conditions of the chemical purity were as in Example 8. The analytical conditions of the optical purity were as follows; column: CHIRALPAK AS (Daicel Chemical Industries, Ltd.), mobile phase: isopropanol/hexane = 3/7, flow rate: 0.5 mL/min, detection wavelength: 210 nm, column temperature: 30°C.

### (Example 10) Method for preparing L-5-methyl-5-tert-butylthiomethylhydantoin

L-5-Chloromethyl-5-methylhydantoin (10.00 g, 61.51 mmol) was dissolved in a 10 wt% sodium hydroxide solution (100 g) and then tert-butylmercaptan (10.4 mL, 92.27 mmol) was added. The solution was stirred under nitrogen atmosphere at 50°C for 3 hours. The reaction mixture was spontaneously cooled to room temperature and then washed with toluene (100 mL × 3). The reaction mixture was adjusted with concentrated hydrochloric acid to pH 1 to 2. The generated white solid was separated by filtration, washed with water (100 mL × 3), and then dried under reduced pressure to yield a white solid of the titled compound (7.79 g, a yield of 58.6%, the analytical conditions were as in Reference Example 1).
¹H NMR (300 MHz, CD₃OD), δ: 2.89 (s, 2H), 1.46 (s, 3H), 1.31 (s, 9H).

### (Example 11) Method for preparing L-5-methyl-5-benzylthiomethylhydantoin

L-5-Chloromethyl-5-methylhydantoin (10 g, 61.5 mmol) was dissolved in a 10 wt% sodium hydroxide solution (100 g) and then benzylmercaptan (11.5 mL, 98.4 mmol) was added. The solution was stirred under nitrogen atmosphere at 50°C for 3 hours. The reaction mixture was spontaneously cooled to room temperature and was then washed with toluene (20 mL × 2). The reaction mixture was adjusted to pH 2.0 with concentrated hydrochloric acid. The resulting white solid was separated by filtration, washed with water, and then dried under reduced pressure to yield a white solid of the titled compound (14.82 g, a yield of 96.3%, the analytical conditions were as in Reference Example 1).
¹H NMR (300 MHz, CDCl₃), δ: 8.61 (s, 1H), 7.34-7.26 (m, 5H), 6.06 (s, 1H), 3.76 (d, 1H), 3.72 (d, 1H), 2.79 (d, 1H), 2.72 (d, 1H), 1.45 (s, 3H).

### (Example 12) Method for preparing L-1-N-acetyl-5-acetylthiomethyl-5-methylhydantoin

L-5-Chloromethyl-5-methylhydantoin (5.88 g, 34.22 mmol, a chemical purity of 94.6%, an optical purity of 98.3% ee) was dissolved in dimethylsulfoxide (137 g) under nitrogen atmosphere, and then potassium thioacetate (14.05 g, 123.0 mmol) was added and reacted at 70°C for 21 hours. The solution was spontaneously cooled to room temperature and then adjusted to pH 6 to 7 with a 10% hydrochloric acid solution. The solution was extracted with ethyl acetate. The resulting organic phase was dried over anhydrous magnesium sulfate. The ethyl acetate was evaporated under reduced pressure to yield an orange solid (12.45 g). Analysis by HPLC (the analytical conditions were as in Reference Example 1) showed that the orange solid contained 5.48 g of the titled compound (22.43 mmol, a chemical purity of 44%, a yield of 65.5%).
¹H NMR (300 MHz, CDCl₃), δ: 8.61 (s, 1H), 7.27 (s, 1H), 3.92 (d, 1H), 3.48 (d, 1H), 2.54 (s, 3H), 2.33 (s, 3H), 1.80 (s, 3H).

### (Example 13) Method for purifying L-1-N-acetyl-5-acethylthiomethyl-5-methylhydantoin

L-1-N-Acetyl-5-acetylthiomethyl-5-chloromethylhydantoin (5.48 g) obtained in the above Example was dissolved in ethyl acetate (22 g) and then heated to 60°C. Hexane was slowly dropped until the white turbidity disappeared. The amount of hexane used was 39.2 g. The solution was spontaneously cooled to room temperature and was then stirred for 20 hours. Then, the solution was gradually cooled to -20°C and was stirred at -20°C for 6 hours. Precipitated white crystals were separated by filtration and washed with 39.2 g of a hexane solution containing 1% ethyl acetate to obtain the titled compound with a yield of 78.3%, a chemical purity of 87.1%, and an optical purity of 99.1% ee. The analytical conditions of the chemical purity were as in Reference Example 1. The optical purity was determined by HPLC [column: CHIRALCEL OD-H (Daicel Chemical Industries, Ltd.), mobile phase: hexane/isopropanol = 9/1, flow rate: 1.0 mL/min, detection wavelength: 210 nm, column temperature: 30°C].

### (Example 14) Method for preparing L-2-benzylthiomethylalanine

L-5-Benzylthiomethyl-5-methylhydantoin (5.0 g, 20 mmol) was dissolved in a 2 N sodium hydroxide solution (40 g) and stirred for 2 days at 100°C. The solution was spontaneously cooled to room temperature and then adjusted to pH 7 with concentrated hydrochloric acid. Precipitated white crystals were separated by filtration and washed with water several times. After drying under reduced pressure, the white crystalline product was analyzed by ¹H NMR and HPLC (the analytical conditions were as in Reference Example 1). The results of the analyses showed that the resulting white crystal was the target compound (a yield of 78%).
¹H NMR (300 MHz, NaOD/D₂O), δ: 7.28-7.08 (m, 5H), 3.71 (s, 2H), 2.88 (d, 1H), 2.72 (d, 1H), 1.31 (s, 3H).

### (Example 15) Method for preparing L-2-tert-butylthiomethylalanine

L-5-tert-Butylthiomethyl-5-methylhydantoin (1.00 g, 4.62 mmol) was dissolved in a 10 wt% sodium hydroxide solution (14.97 g) and stirred for 3 days at 110°C. The solution was spontaneously cooled to room temperature and then adjusted to pH 6.5 with 4 M hydrochloric acid. A white precipitate was removed by filtration. The filtrate was concentrated to yield white crystals. The white crystals were separated by filtration and washed with water several times. After drying under reduced pressure, the white crystalline product was analyzed by ¹H NMR and HPLC (the analytical conditions were as in Reference Example 1). The results of the analyses showed that the resulting white crystals were the target compound (a yield of 78.8%).
¹H NMR (300 MHz, NaOD/D₂O), δ: 2.65 (d, 1H), 2.63 (d, 1H), 1.25 (s, 12H).

### (Example 16) Method for preparing L-2-benzylthiomethylalanine

L-5-Chloromethyl-5-methylhydantoin (200 mg, 1.23 mmol) was dissolved in a 10 wt% sodium hydroxide solution (2.0 g). Then, benzyl mercaptan (0.23 mL, 2.0 mmol) was added and the mixture was stirred under nitrogen atmosphere at 50°C for 5 hours. The reaction temperature was raised to 100°C and the mixture was further stirred for 26 hours. After the addition of a 30 wt% sodium hydroxide solution (1.0 g), the mixture was further stirred at 100°C for 24 hours. Analysis by HPLC showed that the titled compound (a yield of 78.0%) was prepared.

### (Example 17) Method for preparing L-2-tert-butylthiomethylalanine

L-5-Chloromethyl-5-methylhydantoin (501.8 mg, 3.08 mmol) was dissolved in a 10 wt% sodium hydroxide solution (5.03 g). Then, tert-butyl mercaptan (0.55 mL, 4.87 mmol) was added and the mixture was stirred under nitrogen atmosphere at 50°C for 5.5 hours. The reaction temperature was raised to 100°C and the solution was further stirred for 21 hours. After the addition of a 10 wt% sodium hydroxide solution (5.00 g), the solution was further stirred for 2 days. After the reaction, the solution was spontaneously cooled to room temperature and then adjusted to pH 6.5 with a 4 M hydrochloric acid solution to separate any white precipitate. After removing the white precipitate by filtration, the filtrate was concentrated to yield white crystals. The white crystals were separated by filtration and then washed with water several times. The white crystals were dried under reduced pressure and analyzed by ¹H NMR and HPLC (the analytical conditions were as in Reference Example 1). The results of the analyses indicated that the resulting white crystals were the titled compound (a yield of 53.1%).

### (Example 18) Method for preparing L-α-methylcysteine hydrochloride

L-β-Benzylthiomethylalanie (1 g, 4.44 mmol) was dissolved in liquid ammonia (50 mL) under nitrogen atmosphere, and then metallic sodium was slowly added at - 78°C. When the color of the solution changed to blue, the temperature of the solution was raised until the reaction solution was refluxed. The solution was stirred for 30 minutes, while metallic sodium was added if the blue color of the solution disappeared. Then, ammonium chloride was added until the color of the solution changed from blue to white, and the solution was heated to remove the liquid ammonia by vaporization. The solution was adjusted to pH 1 to 2 with a 2 N hydrochloric acid solution, and the water was evaporated under reduced pressure. The resulting white solid was further dried overnight under reduced pressure. The white solid was slurried with THF (13 mL) and ethyl alcohol (6.5 mL), and the slurry was stirred for about 10 minutes. Insoluble matter was removed by filtration. The mother liquor was concentrated and the resulting white solid was further dried under reduced pressure for several hours. After crystallization using ethyl alcohol and toluene, the resulting white crystals (473 mg) were analyzed by ¹H NMR and HPLC [column: CAPCELL PAK SCX (Shiseido Co., Ltd.), mobile phase: potassium dihydrogenphosphate-phosphoric acid solution (pH 2.0)/acetonitrile = 95/5, flow rate: 0.3 mL/min, detection wavelength: 210 nm, column temperature: 30°C]. The results of the analyses indicated that the white crystals were the target compound (a yield of 62.6%). The optical rotation was [α]^{D}₂₀ = 8.77 (c, 1.15 in H₂O). Since the signs were identical to values disclosed in publications (Tetrahedron, 1993, 49:2131-2138 and International Publication No. WO98/38177), the target L-form configuration was identified.
¹H NMR (300 MHz, D₂O), δ: 3.18 (d, 1H), 2.89 (d, 1H), 1.6 (s, 3H).

### (Example 19) Method for preparing L-α-methylcysteine hydrochloride

L-β-tert-Butylthiomethylalanine (433.9 mg, 2.27 mmol) was dissolved in concentrated hydrochloric acid (9 mL), and the solution was refluxed for 4 hours under nitrogen atmosphere. After the solution was spontaneously cooled to room temperature, the water was evaporated under reduced pressure. The resulting white solid was further dried under reduced pressure by using a vacuum pump. The white solid was slurried with THF (6 mL) and ethyl alcohol (2 mL), and the slurry was stirred for about 10 minutes. Insoluble matter was removed by filtration. The mother liquor was concentrated and the resulting white solid was dried under reduced pressure for several hours. After crystallizing from ethyl alcohol and toluene, the resulting white crystals (229 mg) were analyzed by ¹H NMR and HPLC (the analytical conditions were as in Example 18). The results of the analyses showed that the white crystals were a hydrochloride of the target compound (a yield of 58.7%).

### (Example 20) Method for preparing L-α-methylcysteine hydrochloride

L-5-Mercaptomethyl-5-methylhydantoin (1.0 g, a purity of 89.2 wt%, an optical purity of 98.6% ee, 5.57 mmol) was dissolved in concentrated hydrochloric acid (20 g), and the solution was refluxed for 60 hours under nitrogen atmosphere. After the solution was spontaneously cooled to room temperature, the hydrochloric acid solution was evaporated under reduced pressure. THF (50 mL), a 1 N hydrochloric acid solution (5 mL), and triphenylphosphine (2.73 g) were added, and the solution was reacted under nitrogen atmosphere at 50°C for 2 hours. The solution was spontaneously cooled to room temperature, and then concentrated to approximately one fifth the volume of the original solution. The residue was then washed with ethyl acetate (50 mL × 3) to remove triphenylphosphine, triphenylphosphine oxide, unreacted hydantoin, and other impurities. The aqueous phase was concentrated under reduced pressure, and the resulting white solid was further dried under reduced pressure overnight. The white solid was slurried with THF (15.6 mL) and ethyl alcohol (7.8 mL), and the slurry was stirred for about 10 minutes. Insoluble matter was removed by filtration. The mother liquor was concentrated to yield a white solid of the titled compound (879 mg). Analysis by ¹H NMR and HPLC (the analytical conditions were as in Example 18) confirmed that the white solid was the target compound (a yield of 92.3%).

### (Example 21) Method for preparing L-α-methylcysteine hydrochloride

L-5-Mercaptomethyl-5-methylhydantoin (500 mg, a purity of 89.2 wt%, an optical purity of 98.6% ee, 2.78 mmol) was dissolved in a 30 wt% sodium hydroxide solution (5 g), and the solution was refluxed for 36 hours under nitrogen atmosphere. The solution was spontaneously cooled to room temperature and then adjusted to pH 1 with concentrated hydrochloric acid. Then, the water was evaporated under reduced pressure. THF (30 mL), a 1N hydrochloric acid solution (3 mL), and triphenylphosphine (1.57 g) were added and the solution was reacted under nitrogen atmosphere at 50°C for 2 hours. The solution was spontaneously cooled to room temperature. Next, the solution was concentrated to approximately one-fifth the volume of the original solution and then washed with ethyl acetate (50 mL × 3) to remove triphenylphosphine, triphenylphosphine oxide, unreacted hydantoin, and other impurities. The aqueous phase was concentrated under reduced pressure, and the resulting white solid was further dried under reduced pressure overnight. The white solid was slurried with THF (9.3 mL) and ethyl alcohol (4.7 mL), and the slurry was stirred for about 10 minutes. Insoluble matter was removed by filtration. The mother liquor was concentrated to yield a white solid of the titled compound (348 mg). Analysis by ¹H NMR and HPLC (the analytical conditions were as in Example 18) confirmed that the white solid was the target compound (a chemical purity of 74.1%, a yield of 48.0%).

### (Example 22) Method for preparing L-α-methylcysteine hydrochloride

L-1-N-Acetyl-5-acetylthiomethyl-5-methylhydantoin (3.4 g, 13.9 mmol) was dissolved in concentrated hydrochloric acid (34 g), and the solution was refluxed for 20 hours under nitrogen atmosphere. Concentrated hydrochloric acid (34 g) was added and the solution was further reacted for 30 hours. After the solution was spontaneously cooled to room temperature, the hydrochloric acid solution was evaporated under reduced pressure, and then THF (50 mL), 1 N hydrochloric acid solution (5 mL), and triphenylphosphine (2.17 g) were added. The solution was reacted under nitrogen atmosphere at 50°C for 2 hours. The solution was spontaneously cooled to room temperature. Then, the solution was concentrated to approximately one fifth the volume of the original solution, and then washed with ethyl acetate to remove triphenylphosphine, triphenylphosphine oxide, unreacted hydantoin, and other impurities. The aqueous phase was concentrated under reduced pressure, and the resulting white solid was further dried under reduced pressure overnight. The white solid was slurried with THF (18 mL) and ethyl alcohol (6 mL), and the slurry was stirred for about 10 minutes. Insoluble matter was removed by filtration. The mother liquor was concentrated, and the resulting white solid was further dried under reduced pressure for several hours. After crystallization using ethyl alcohol and toluene, the resulting white crystals (1.02 g) were analyzed by ¹H NMR and HPLC (the analytical conditions were as in Example 18). The results of the analyses showed that the white crystals were the target compound (a yield of 43.0%).

### (Example 23) Method for preparing L-α-methylcysteine hydrochloride

L-5-tert-Butylthiomethyl-5-methylhydantoin (202.3 mg, 0.935 mmol) was dissolved in concentrated hydrochloric acid (4 mL), and the solution was stirred under nitrogen atmosphere at 110°C for 26 hours. After the solution was spontaneously cooled to room temperature, the water was evaporated under reduced pressure. Analysis by ¹H NMR and HPLC (the analytical conditions were as in Example 18) of the resulting residue confirmed that the yield of L-α-methylcysteine was 42.5% and the yield of L-5-mercaptomethyl-5-methylhydantoin was 53.7%.

### (Example 24) Method for preparing L-α-methylcysteine hydrochloride

L-5-tert-Butylthiomethyl-5-methylhydantoin (202.5 mg, 0.936 mmol) was dissolved in concentrated hydrochloric acid (4 mL) and the solution was stirred in an autoclave at 150°C for 26 hours. After the solution was spontaneously cooled to room temperature, the water was evaporated under reduced pressure. Analysis by HPLC (the analytical conditions were as in Example 18) of the resulting residue confirmed that the yield of L-α-methylcysteine was 46.3% and the yield of the disulfide of L-α-methylcysteine was 53.7%.

### (Reference Example 2) Method for determining the optical purity of L-α-methylcysteine hydrochloride

L-α-Methylcysteine hydrochloride (74.9 mg, 0.44 mmol) prepared as in Example 18 was dissolved in water (3 mL), and sodium hydrogencarbonate (197.7 g) and then ethyl alcohol (3 mL) were added. After nitrogen purge, benzyl chloroformate (0.17 mL, 1.10 mmol) was added and stirred at a room temperature for 2 days. The reaction solution was adjusted to pH 1.9 with concentrated hydrochloric acid. After extraction with ethyl acetate, the solution was dried over anhydrous sodium sulfate and the solvent was evaporated under reduced pressure. The residue was purified by PTLC (hexane/ethyl acetate = 1/1 and a small amount of acetic acid). Analysis by ¹H NMR identified the product as the target compound (106 mg, a yield of 60%). Analysis by HPLC [column: CHIRALCEL OD-RH (Daicel Chemical Industries, Ltd.), mobile phase: potassium dihydrogenphosphate-phosphoric acid solution (pH 2.0)/acetonitrile = 6/4, flow rate: 1.0 mL/min, detection wavelength: 210 nm, column temperature: 30°C, retention time: 19.15 minutes (D) and 22.92 minutes (L)] showed an optical purity of 98.6% ee.
¹H NMR (300 MHz, D₂O), δ: 7.30-7.40 (m, 10H), 5.22 (s, 2H), 5.10 (s, 2H), 3.60 (s, 2H), 1.63 (s, 3H).

### Industrial Applicability

According to the present invention, an L-α-methylcysteine derivative and its salt useful as a drug intermediate can be easily prepared from a cheap and readily available material.

## Claims

1. A method for preparing an L-5-halomethyl-5-methylhydantoin represented by Formula (2): 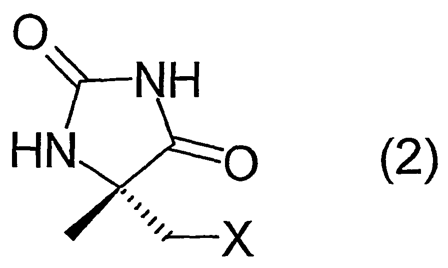 (wherein X denotes a halogen atom), comprising:
D-stereoselectively hydrolyzing a racemic 5-halomethyl-5-methylhydantoin represented by Formula (1): 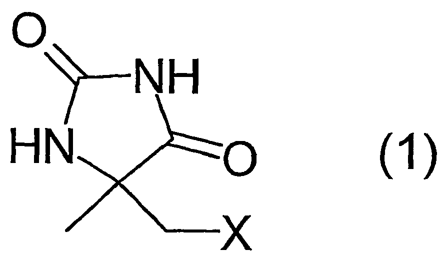 (wherein X is the same as above), with hydantoinase.

2. The method according to claim 1, wherein X is a chlorine atom.

3. The method according to claim 1 or 2, wherein the hydantoinase is derived from a microorganism belonging to Agrobacterium, Bacillus, Pseudomonas, or Rhizobium.

4. The method according to claim 1 or 2, wherein the hydantoinase is derived from Agrobacterium sp. strain KNK712 (FERM BP-1900), Bacillus sp. strain KNK245 (FERM BP-4863), Pseudomonas putida IFO 12996, Pseudomonas sp. strain KNK003A (FERM BP-3181), or Rhizobium sp. strain KNK1415 (FERM BP-4419).

5. The method according to claim 1 or 2, wherein the hydantoinase is derived from a transformed microorganism selected from the group consisting of Escherichia coli HB101 pTH104 (FERM BP-4864), Escherichia coli HB101 pAH1043 (FERM BP-4865), and Escherichia coli HB101 pPHD301 (FERM BP-4866).

6. A method for preparing an L-5-halomethyl-5-methylhydantoin, comprising:
crystallizing an L-5-halomethyl-5-methylhydantoin represented by Formula (2) with at least one solvent selected from the group consisting of ethyl acetate, methyl alcohol, ethyl alcohol, propyl alcohol, isopropyl alcohol, n-butyl alcohol, sec-butyl alcohol, tert-butyl alcohol, acetone, tetrahydrofuran, and acetonitrile to improve the optical purity.

7. The method according to claim 6, wherein at least one poor solvent selected from the group consisting of benzene, toluene, hexane, heptane, and cyclohexane is simultaneously used.

8. The method according to claim 6 or 7, wherein the L-5-halomethyl-5-methylhydantoin prepared by the method of claim 1 is used for the crystallization.

9. A method for preparing an L-5-methyl-5-thiomethylhydantoin represented by Formula (3): 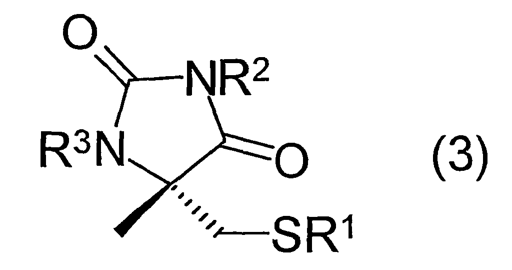 (wherein R¹ denotes a hydrogen atom, an alkyl group having 1 to 20 carbon atoms which may be linear, branched, or cyclic, a substituted or unsubstituted benzyl group, or an alkanoyl group having 1 to 20 carbon atoms; and each of R² and R³ independently denotes a hydrogen atom, an alkanoyl group having 1 to 20 carbon atoms, or an alkoxycarbonyl group having 1 to 20 carbon atoms, and may be the same or different), comprising:
reacting an L-5-halomethyl-5-methylhydantoin represented by Formula (2) with a sulfurizing agent.

10. The method according to claim 9, wherein the L-5-methyl-5-thiomethylhydantoin is prepared by reacting an L-5-halomethyl-5-methylhydantoin prepared by the method according to any one of claims 1 to 5 with a sulfurizing agent.

11. The method according to claim 9 or 10, wherein the sulfurizing agent is selected from the group consisting of an alkali metal hydrosulfide, an alkaline-earth metal hydrosulfide, ammonium hydrosulfide, an alkyl mercaptan, an aralkyl mercaptan, and thioacetic acid or its alkali metal salt.

12. The method according to claim 9 or 10, wherein the sulfurizing agent is selected from the group of consisting of sodium hydrosulfide, potassium hydrosulfide, potassium thioacetate, tert-butyl mercaptan, benzyl mercaptan, and p-methoxybenzyl mercaptan.

13. A method for preparing an L-α-methylcysteine derivative or its salt represented by Formula (4): 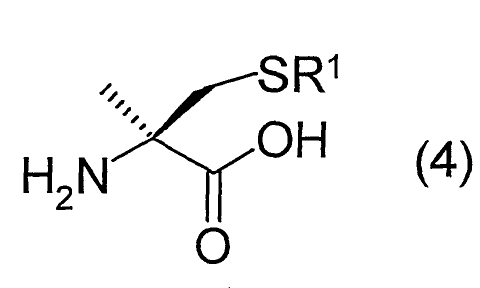 (wherein R¹ denotes a hydrogen atom; an alkyl group having 1 to 20 carbon atoms which may be linear, branched, or cyclic, a substituted or unsubstituted benzyl group, an alkanoyl group having 1 to 20 carbon atoms, or an alkoxycarbonyl group having 1 to 20 carbon atoms), comprising:
hydrolyzing an L-5-methyl-5-thiomethylhydantoin represented by Formula (3) with an acid or alkali; and optionally deprotecting a nitrogen atom and/or a sulfur atom, wherein the L-5-methyl-5-thiomethylhydantoin is prepared by the method according to any one of claims 9 to 12.

14. The method according to claim 13, wherein the hydrolysis is performed with at least one acid selected from the group consisting of hydrochloric acid, sulfuric acid, nitric acid, hydrobromic acid, acetic acid, and trifluoroacetic acid.

15. The method according to claim 13, wherein the hydrolysis is performed with at least one alkali selected from the group consisting of lithium hydroxide, sodium hydroxide, potassium hydroxide, calcium hydroxide, barium hydroxide, sodium carbonate, and potassium carbonate.

16. A method for preparing an L-α-methylcysteine derivative or its salt represented by Formula (4): 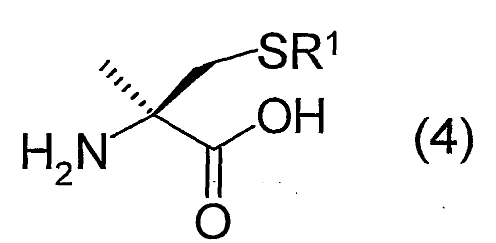 (wherein R¹ denotes a hydrogen atom, an alkyl group having 1 to 20 carbon atoms which may be linear, branched, or cyclic, a substituted or unsubstituted benzyl group; an alkanoyl group having 1 to 20 carbon atoms, or an alkoxycarbonyl group having 1 to 20 carbon atoms), comprising:
reacting an L-5-halomethyl-5-methylhydantoin represented by Formula (2) and a sulfurizing agent in an alkaline solution, the sulfurization and hydrolysis being performed in a one-pot; and optionally deprotecting a sulfur atom.

17. The method according to claim 16, wherein the L-5-halomethyl-5-methylhydantoin prepared by the methods according to any one of claims 1 to 5 is used.

18. The method according to any one of claims 13 to 16, wherein L-α-methylcysteine or its salt having a hydrogen atom as R¹ in Formula (4) is prepared by cleavage a disulfide bond of a disulfide by-product with a reducing agent.

19. The method according to claim 18, wherein the reducing agent is a trialkylphosphine or triarylphosphine.

20. The method according to claim 18, wherein the reducing agent is triphenylphosphine.

21. An L-5-halomethyl-5-methylhydantoin represented by Formula (2): 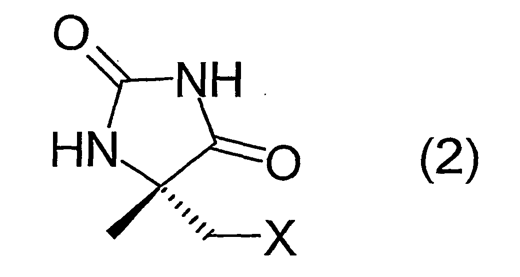 (wherein X denotes a halogen atom).

22. The compound according to claim 21, wherein X is a chlorine atom.

23. An L-5-methyl-5-thiomethylhydantoin represented by Formula (5): 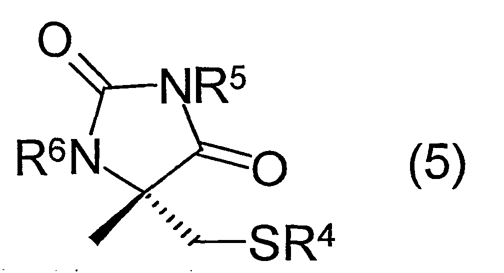 (wherein R⁴ denotes an alkyl group having 1 to 20 carbon atoms which may be linear, secondary, or cyclic, a substituted or unsubstituted benzyl group, an alkanoyl group having 1 to 20 carbon atoms, or an alkoxycarbonyl group having 1 to 20 carbon atoms; and each of R⁵ and R⁶ independently denotes a hydrogen atom or an alkanoyl group having 1 to 20 carbon atoms and may be the same or different).

24. The compound according to claim 23, wherein R⁵ and R⁶ are hydrogen atoms, and R⁴ is a benzyl group or a p-methoxybenzyl group.

25. The compound according to claim 22, wherein R⁴ and R⁶ are acetyl groups and R⁵ is a hydrogen atom.

26. The compound according to claim 23, wherein all R⁴, R⁵, and R⁶ are acetyl groups.

27. An L-α-methylcysteine derivative or its salt with an acid represented by Formula (6): 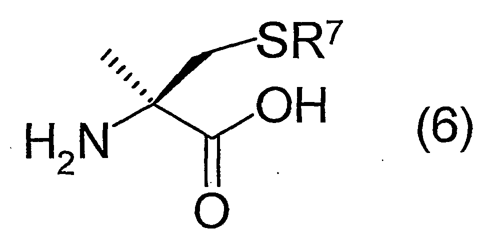 (wherein R⁷ is a substituted or unsubstituted benzyl group, an alkanoyl group having 1 to 20 carbon atoms, or an alkoxycarbonyl group having 1 to 20 carbon atoms).

28. The compound according to claim 27, wherein R⁷ is a benzyl group, a p-methoxybenzyl group, or an acetyl group.

29. The compound according to claim 27, wherein the acid is hydrochloric acid, hydrobromic acid, sulfuric acid, p-toluenesulfonic acid, or comphorsulfonic acid.
